# EUROPÄISCHE PATENTSCHRIFT

(11) **EP 1 427 692 B1**
(45) Veröffentlichungstag und Bekanntmachung des Hinweises auf die Patenterteilung: **14.03.2007**
(21) Anmeldenummer: 02774555.3
(22) Anmeldetag: 03.09.2002
(51) Int. Cl.: C07C 67/08, C07C 69/54

(54) **VERFAHREN ZUR HERSTELLUNG VON (METH)ACRYLSAEUREESTERN**
METHOD FOR PRODUCING (METH)ACRYLIC ACID ESTERS
PROCEDE POUR PREPARER DES ESTERS D'ACIDE (METH)ACRYLIQUE

(30) Priorität: 10.09.2001 DE 10144490
(43) Veröffentlichungstag der Anmeldung: 16.06.2004
(73) Patentinhaber: BASF Aktiengesellschaft, 67056 Ludwigshafen (DE)
(72) Erfinder: MARTAN, Hans, 67227 Frankenthal (DE); NESTLER, Gerhard, A-1070 Wien (AT)
(86) Internationale Anmeldenummer: PCT/EP2002/009814
(87) Internationale Veröffentlichungsnummer: WO 2003/022793

(56) Entgegenhaltungen:
- DE-A- 19 952 449
- US-A- 5 187 309

## Beschreibung

Die vorliegende Erfindung betrifft ein Verfahren zur Herstellung von (Meth)acrylsäureestern durch Umsetzung von Alkoholen mit einer weitgehend wasserfreien Roh-(Meth)acrylsäure in Gegenwart mindestens eines stark sauren Katalysators und eines Inhibitors oder Inhibitorgemisches in homogener, flüssiger Phase, in dem trotz Verwendung von Roh-(Meth)acrylsäure hochreine (Meth)acrylsäureester in hoher Ausbeute gewonnen werden können.

Unter Roh-(Meth)acrylsäure wird hier das (meth)acrylsäurehaltige Gemisch verstanden, das nach Absorption der Reaktionsgase der Propan/Propen/Acrolein- beziehungsweise Isobutan/Isobuten/Methacrolein-Oxidation in einem Absorptionsmittel und anschließender Abtrennung des Absorptionsmittels anfällt beziehungsweise das durch fraktionierende Kondensation der Reaktionsgase gewonnen wird.

Die erhaltenen (Meth)acrylsäureester werden auch bei Einsatz von Roh-(Meth)acrylsäure in hoher Reinheit und im wesentlichen frei von Ethern, gesättigten Estern, wie z.B. Acetate und Propionate, aldehydischen und anderen carbonylhaltigen, sowie hochsiedenden Nebenkoinponenten erhalten.

Die auf Basis von (Meth)acrylsäureestern hergestellten Polymerisate bzw. Copolymerisate haben allgemein in Form von Polymerdispersionen eine große wirtschaftliche Bedeutung, z.B. als Klebstoffe, Anstrichmittel oder Textil-, Leder-, und Papierhilfsmittel. Für Anwendungen, besonders im Lebensmittel- oder Kosmetikbereich sollten die Polymerdispersionen weitgehend frei sein von flüchtigen Verunreinigungen, wie niederen Aldehyden, besonders C_{I}-C₄-Aldehyden, wie Formaldehyd, Acetaldehyd, Propionaldehyd, Acrolein, Methacrolein und iso-Butyraldehyd, oder Furfural, Benzaldehyd und andere carbonylhaltigen Nebenkomponenten, wie z.B. Aceton, sowie Essigsäure, Propionsäure, Protoanemonin (5-Methylen-2(5H)-furanon), sowie Ether des eingesetzten Alkohols, Essigsäure-, Propionsäure-, Maleinsäureester und Michael-Additionsprodukte aus dem eingesetzten Alkohol und der (Meth)acrylsäure (Alkoxy- und Acryloxypropionsäureester und deren höheren Homologen), wobei die Essig-, Propion- und Maleinsäureester durch Veresterung der in der Roh-(Meth)acrylsäure vorhandenen Essig-, Propion- und Maleinsäure entstehen.

Aus ökologischen und ökonomischen Gründen werden daher äußerst reine (Meth)acrylsäureester als Ausgangsstoffe gefordert, so daß unerwünschte Begleitstoffe nicht z.B. durch aufwendige chemische oder physikalische Desodorierung nachträglich entfernt werden müssen.

Die Herstellung von niederen (Meth)acrylsäureestern durch Veresterung von (Meth)acrylsäure mit niederen Alkoholen in Gegenwart von starken Säuren ist allgemein bekannt. Als (Meth)acrylsäure wird dabei in der Regel reine oder vorgereinigte (Meth)acrylsäure eingesetzt, siehe z.B. Chem Systems, Acrylic Acid/Acrylates 96/97-8, Nov. 1997, Seite 24.

Unter reiner bzw. vorgereinigter (Meth)acrylsäure wird allgemein (Meth)acrylsäure verstanden, deren Reinheit mind. 99,5 Gew.-% beträgt und die im wesentlichen frei von aldehydischen, anderen carbonylhaltigen und hochsiedenden Komponenten ist. Die Reinigung dieser (Meth)acrylsäure erfolgt in der Regel durch mehrstufige Kristallisation oder gegebenenfalls durch chemische Behandlung mit einem Aldehydfänger und Destillation.

Da bekanntlich der Bildung des Esters aus (Meth)acrylsäure und Alkohol eine Gleichgewichtsreaktion zugrunde liegt, wird, um wirtschaftliche Umsätze zu erzielen, in der Regel ein Einsatzstoff im Überschuss eingesetzt und/oder das gebildete Veresterungswasser und/oder der Zielester aus dem Gleichgewicht entfernt. Die Beeinflussung des Veresterungsgleichgewichts durch den Einsatz eines Alkoholüberschusses ist jedoch nachteilig, da dadurch u.a. die Bildung von Ethern aus den Ausgangsalkoholen und von Michael-Additionsprodukten gefördert wird (siehe z.B. US 4 280 010, Spalte 1).

Da (Meth)acrylverbindungen leicht zu unerwünschter Polymerisation neigen, besonders unter Einwirkung von Hitze, werden allgemein große Anstrengungen unternommen, um die Bildung von Polymerisat während der Veresterung und Isolierung des Zielesters zu vermeiden.

In der Regel führt diese Polymerisatbildung nämlich zu einer Belegung der Reaktorwände, Wärmetauscherflächen und Kolonnenböden (Fouling) sowie zu einem Verstopfen von Leitungen, Pumpen, Ventilen etc. (EP-A 522 709, S. 2, Z. 9 - 18; US 5 171 888, Spalte 1, Z. 19 - 38). Die Folge sind teure Abstellungen und aufwendige Reinigungsoperationen, wie z.B. das in der DE-A 195 36 179 beschriebene Kochen mit basischen Lösungen, die anschließend aufwendig entsorgt werden müssen.

W. Bauer jr. beschreibt in der "Encyclopedia of Chemical Technology", Kirk-Othmer, 4. Ed., 1994, Vol. 1, S. 301 - 302, ein Verfahren, das darin besteht, daß die Veresterung in Gegenwart von Schwefelsäure bei 80 °C durchgeführt wird, der Zielester, das Reaktionswasser und der überschüssige Alkohol destillativ abgetrennt werden, ein Teil des Destillats als Rücklauf in die dem Reaktor aufgesetzte Kolonne zurückgeführt und der Rest einer Waschkolonne zugeführt wird, die gewaschene feuchte Esterphase in einer Destillationskolonne unter hohem Rücklauf entwässert wird und in einem weiteren Destillationsschritt der Zielester aus dem wasserfreien Sumpfprodukt isoliert wird. Das dabei wieder anfallende Sumpfprodukt wird in einer weiteren Destillation in eine Wertproduktfraktion, die in die Esterdestillation zurückgeführt wird, und eine Sumpffraktion, die ausgeschleust wird, aufgetrennt. Aus dem Reaktor wird kontinuierlich ein Strom entnommen und in einem Destillationsschritt von den Hochsiedern befreit, wobei das Destillat in die Veresterung zurückgeführt wird. Alle anfallenden Wasserphasen werden vereinigt und das darin gelöste Alkanol destillativ zurückgewonnen.

Dieses Verfahren erfordert zumindest eine vorgereinigte Acrylsäure, da sonst leichtflüchtige Essig- und Propionsäureester per Rücklauf in der dem Reaktor aufgesetzten Kolonne aufgepegelt werden.

In Ullmann's Encyclopedia of Industrial Chemistry, 5. Ed., 1985, Vol. A1, S. 167 - 168 wird darauf hingewiesen, daß bei der Herstellung von niederen Estern (Methyl- und Ethylacrylat) Kationenaustauscherharze als Katalysatoren vorteilhafter sind als Schwefelsäure, die wiederum bei der Herstellung von höheren Ester bevorzugt wird. Das beschriebene Verfahren besteht darin, daß die Veresterung in einem Kationenaustauscherfestbett bei 60 - 80 °C durchgeführt und der flüssige Reaktoraustrag destillativ, vergleichbar dem oben beschriebenen Verfahren, aufgearbeitet wird.

US 5 187 309 schlägt ein Veresterungsverfahren vor, das von wässriger Acrylsäure (30 - 50 % Wasser) ausgeht. Die Veresterung erfolgt bei 50 - 110 °C in Gegenwart einer Säure als Katalysator und das Veresterungsgemisch wird gemeinsam mit der wässrigen Acrylsäure einer Destillationseinheit zugeführt. Als Sumpfprodukt fällt Acrylsäure an, am Kopf der Kolonne wird Wasser, Alkanol und Acrylester kondensiert und anschließend weiter aufgearbeitet. Die Acrylsäure wird direkt in die Veresterung rückgeführt bzw. ein Teilstrom vorher destillativ gereinigt (Abtrennung von Hochsiedern und Polymerisat). Das esterhaltige Gemisch wird mit Wasser alkanolfrei gewaschen und anschließend daraus destillativ der Zielester isoliert.

Der Nachteil des Verfahrens besteht darin, daß hohe Verluste an Wertprodukt (ca. 5,9 % bzgl. Esterproduktion, s. Bsp. 1, Tab. 1, Stream F9) entstehen, hohe Rückstandsmengen anfallen (ca. 4,5 % bezüglich Zielester, Stream F4) und ein hoher Energiebedarf notwendig ist, da große Wassermengen (Veresterungswasser und Wasser der wässrigen Acrylsäure; ca. 3 Mol/Mol Ester), verdampft werden müssen. Hoch sind auch die Acrylsäureverluste im Waschwasser (ca. 1 % bezüglich Ester, Stream F7).

US 3 875 212 schlägt bei der Veresterung den Einsatz eines organischen Lösungsmittels mit einem Siedepunkt von 100 - 160 °C vor, um das Veresterungswasser als Azeotrop aus dem Reaktionsgemisch zu entfernen. Auf diese Weise soll die Verwendung von Schwefelsäure als Katalysator ermöglicht werden, die Einstellung einer konstanten Katalysatorkonzentration erleichtert werden und ein besser handhabbares Polymerisat entstehen. Das Verfahren besteht darin, daß die Acrylsäure mit dem Alkanol in Gegenwart von Schwefelsäure und des Lösungsmittels verestert wird, und das.Veresterungswasser kontinuierlich destillativ als Azeotrop mit dem Acrylester und dem Lösungsmittel abgetrennt wird. Der Reaktoraustrag zerfällt in eine wässrige Phase und eine organische Phase, die einer Wasserdampfdestillation unterzogen wird, um den Acrylester und das Lösungsmittel zurückzugewinnen und vom Polymerisat und Hochsiedern abzutrennen. Die wässrige Phase, die die nicht umgesetzte Acrylsäure, die Schwefelsäure und Polymerisat enthält, wird zwecks Abtrennung des Polymerisats filtriert und wieder dem Reaktor zugeführt.

Das Verfahren ist z.B. durch das Erfordernis einer Wasserdampfdestillation und einer Filtration technisch aufwendig und umständlich und hat daher keine wirtschaftliche Bedeutung.

US 4 280 010 beschreibt ein Verfahren zur Herstellung von etherfreien niederen Acrylsäureestern, bei dem die Veresterung bei 80 - 130°C, in Gegenwart von 0,1 - 3 % Schwefelsäure oder 1 - 8 % Sulfonsäure und bei einem Acrylsäure: Alkanol-Verhältnis von 1: 1 - 2 durchgeführt wird. Vorzugsweise wird eine Reaktionstemperatur von 85 - 110 °C vorgeschlagen, da bei höherer Temperatur die Etherbildung und die Polymerisationsneigung überhand nehmen (Spalte 2, Zeilen 53 - 57). Der Zielester, das Veresterungswasser und das Alkanol werden gasförmig ausgeschleust und kondensiert. Das Kondensat zerfällt in zwei Phasen, wobei die Wasserphase abgetrennt und die organische Phase z.T. destillativ aufgearbeitet und z.T. als Rücklauf verwendet wird. Zunächst wird das Alkanol gemeinsam mit dem Zielester als Azeotrop abgetrennt und in den Reaktor zurückgeführt und anschließend der Zielester aus dem Sumpfprodukt isoliert.

Nachteilig bei diesem Verfahren ist, daß reine Acrylsäure eingesetzt werden muß und daß hohe Rückstandsmengen anfallen (3 - 7 %, bzgl. Zielester).

US 4 076 950 beschreibt ein Verfahren zur Herstellung von Acrylsäureestern durch säurekatalysierte Veresterung von Acrylsäure mit Alkanolen und Abtrennung eines Rohesterdestillats, das aus Zielester, Veresterungswasser und überschüssigem Alkanol besteht, in dem im wesentlichen wasserfreier ("substantial anhydrous") Ester dem Reaktor zugeführt wird um ein acrylsäurefreies Rohesterdestillat zu erhalten und um die Zusammensetzung der Ester/Alkanol- und Ester/Wasser-Azeotrope aufrechtzuerhalten. Das Rohesterdestillat muß dann noch einer Feinneutralisation ("polish neutralization") unterworfen werden, bei der die sonst üblichen Phasentrennprobleme und dadurch bedingten Probleme bei der destillativen Aufarbeitung nur noch in geringem Maße auftreten. Das Rohesterdestillat wird bei der Kondensation mit Wasser gewaschen, nach der Phasentrennung wird die organische Esterphase nochmals mit Wasser gewaschen und anschließend destillativ entwässert.

Das Verfahren erfordert die Verwendung von bereits aufgereinigtem Ester zur Aufrechterhaltung der Azeotrope im Reaktor und benötigt zwei Waschvorgänge und mehrere Destillationsstufen und ist daher unwirtschaftlich. Weiterhin erlaubt es nicht die Verwendung von Roh-Acrylsäure.

US 4 464 229 schlägt vor, die Veresterung der Acrylsäure mit einem molaren Unterschuss an Alkanol (1 : 0,5 - 1) durchzuführen. Das Verfahren besteht darin, daß aus dem Veresterungsgemisch, bestehend aus Acrylsäure, Acrylester, Wasser und Alkanol in einer Destillationskolonne der Acrylester, Wasser und Alkanol über Kopf abgetrennt werden, wobei als Rücklauf wasser- und alkanolfreier Acrylester verwendet wird (Sumpf der Leichtsiederabtrennung). Die Rücklaufmenge wird dabei so eingestellt, daß im Destillat ein bestimmter Wassergehalt erreicht wird. Im Falle von Methylacrylat soll dieser 3 - 8 % betragen. Das bedeutet aber, daß pro 100 kg gebildetes Methylacrylat 116 - 477 kg destillativ vorgereinigtes Methylacrylat als Rücklauf benötigt werden.

Nach Abtrennung des Veresterungswasser wird die organische Phase des Destillats, ggf. nach Durchführung einer Extraktion (s. Sp. 4, Z. 21), einer Leichtsiederdestillation zugeführt. Der Sumpf, hauptsächlich aus Acrylsäure bestehend, beträgt mindestens 200 bis 600 % der Menge an frisch zugeführter (Meth)acrylsäure und wird in den Veresterungsreaktor zurückgeführt, vorzugsweise nach destillativer Abtrennung von hochsiedenden Nebenprodukten und Polymerisat. Das Kondensat der Leichtsiederdestillation zerfällt in eine Wasserphase, die ausgeschleust wird, und eine organische Phase, die vollkommen als Rücklauf zurückgeführt wird. Der Sumpf der Leichtsiederkolonne wird teilweise als Rücklauf in der ersten Destillationskolonne verwendet (s.o.) und der Rest in einem weiteren Destillationsschritt zu Acrylester aufgearbeitet. Anfallende Wasserphasen können in einer nicht näher erläuterten Alkoholrückgewinnung aufgearbeitet werden.

Das Verfahren ist aufwendig, es benötigt reine (Meth)acrylsäure als Ausgangsprodukt und es werden große (Meth)acrylsäuremengen und große, destillativ vorgereinigte Estermengen als Rückläufe im Kreis gefahren.

Gesucht wurde ein einfaches und wirtschaftliches Verfahren zur . _ Herstellung von (Meth)acrylsäureestern, in dem von roher (Meth)acrylsäure ausgegangen werden kann, das technisch einfach ist, hohe Ausbeute und hochreine Produkte liefert und bei dem wenig Abfall und möglichst wenig Polymerisat anfällt.

Die Aufgabe wurde gelöst durch ein Verfahren zur Herstellung von (Meth)acrylsäureestern durch Umsetzung von (Meth)acrylsäure mit einem Alkohol in Gegenwart mindestens eines sauren Katalysators und mindestens eines Polymerisationsinhibitors in einer Reaktionszone b), bestehend aus mindestens einem Reaktor b1) mit mindestens einer angeschlossener Destillationseinheit b2), wobei man
- im Reaktor b1) die Temperatur auf 120 bis 150 °C und
- die gewünschte Stöchiometrie (Meth)acrylsäure : Alkohol einstellt,
- das Destillat der Destillationseinheit b2) mit einer Waschflüssigkeit behandelt (Waschschritt c)),
- den Austrag aus dem Waschschritt c) in mindestens eine organische c1) und mindestens zwei wässrige Phasen c2) und c3) trennt,
- die organische Phase c1) teilweise als Rücklauf in der Destillationseinheit b2) und teilweise ohne weitere Waschung in eine destillative Aufreinigung g) des (Meth)acrylsäureesters leitet und
- die wässrigen Phasen c2) und c3) zumindest teilweise in eine Alkoholrückgewinnung d) leitet.

Das erfindungsgemäße Verfahren erfüllt die Aufgabenstellung und besteht darin, daß:
1. als Ausgangsstoff eine Roh-(Meth)acrylsäure eingesetzt werden kann.
2. die Veresterung bei einer Temperatur durchgeführt wird, bei der die Michael-Addukte teilweise zurückgespalten wird.
3. ein Gemisch aus Zielester, Wasser, Alkohol, Alkoxyester und Leichtsieder kontinuierlich aus dem Reaktor b1) über eine angeschlossene Destillationseinheit b2) ausgeschleust wird.
4. Michael-Additionsprodukte in der Alkohol-Rückgewinnungskolonne d) abgetrennt und in den Reaktor b1) zurückgeführt werden.
5. der in der Leichtsiederfraktion der Leichtsiederabtrennung e) enthaltene Zielester in einem weiteren Destillationsschritt (Leichtsieder-Aufarbeitung f) als Sumpfprodukt f2) zurückgewonnen und teilweise in die Leichtsiederabtrennung e) eingeschleust und teilweise als Rücklauf auf die Reaktorkolonne b2) aufgebracht werden kann.
6. das aus dem Reaktor ausgeschleuste Sumpfprodukt b3) max. 10 % Zielester und max. 15 % (Meth)acrylsäure enthält.
7. der ausgeschleuste Rückstand b3) einer Rückstandsaufarbeitung (Stufe 8) und/oder einer Schwefelsäurerückgewinnung zugeführt werden kann.

Das erfindungsgemäße Verfahren kann sowohl zur Herstellung von Methacrylsäure- als auch von Acrylsäureestern eingesetzt werden, bevorzugt zur Herstellung von Acrylsäureestern und sei im folgenden exemplarisch für Acrylsäureester beschrieben.

Die Herstellung der Roh-(Meth)acrylsäure erfolgt auf an sich bekannte Weise in der Regel durch heterogen katalysierte Gasphasenpartialoxidation mindestens eines C₃- beziehungsweise C₄-Vorläufers der (Meth)acrylsäure, wie z.B. Propan, Propen, Acrolein oder Isobutan, Isobuten oder Methacrolein, mit molekularem Sauerstoff bei erhöhter Temperatur.

Dazu wird bei der Herstellung der (Meth)acrylsäure in der Regel das Ausgangsgas mit unter den gewählten Reaktionsbedingungen inerten Gasen wie z.B. Stickstoff (N₂), CO₂, gesättigten C₁-C₆-Kohlenwasserstoffen und/oder Wasserdampf verdünnt und im Gemisch mit molekularem Sauerstoff (O₂) oder einem sauerstoffhaltigen Gas bei erhöhten Temperaturen (üblicherweise 200 bis 450 °C) sowie gegebenenfalls erhöhtem Druck über feste, übergangsmetallische, z.B. Mo und V beziehungsweise Mo, W, Bi und Fe enthaltende Mischoxidkatalysatoren geleitet und oxidativ in (Meth)acrylsäure umgewandelt. Diese Umsetzungen können mehrstufig oder einstufig durchgeführt werden mit je 1, 2 oder mehr Reaktionszonen und/oder Katalysatorschüttungen, die eine von Reaktionszone zu Reaktionszone variable Zusammensetzung und/oder Reaktivität aufweisen können. Beispielhafte Verfahren sind z.B. in DE-A 19 62 431, DE-A 29 43 707, DE-C 12 05 502, EP-A 257 565, EP-A 253 409, DE-A 22 51 364, EP-A 117 146, GB-B 1 450 986 und EP-A 293 224 beschrieben.

Methacrolein kann selbstverständlich auch über Aldolkondensation von Propionaldehyd und Formaldehyd erhalten werden und anschliessend beispielsweise wie oben beschrieben in Methacrylsäure überführt werden.

Bevorzugt wird das erfindungsgemäß eingesetzte, acrylsäurehaltige Produktgasgemisch aus der Partialoxidation von Propan, Propen und/oder Acrolein erhalten.

Das entstehende heiße Reaktionsgasgemisch enthält neben der (kondensierbaren) Acrylsäure und kondensierbaren Nebenkomponenten, z.B. Essigsäure, Propionsäure, Aceton, Acrolein, Allylacrylat, die oben angeführten niederen Aldehyde und Wasser, einen hohen Anteil nicht kondensierbarer Komponenten wie Kohlenoxide, Stickstoff oder Sauerstoff.

Zur Abtrennung der Acrylsäure aus einem solchen Reaktionsgasgemisch sind zahlreiche Verfahren bekannt. So wird z.B. in DE-C 21 36 396 oder DE-A 24 49 780 die Acrylsäure aus den bei der katalytischen Gasphasenoxidation erhaltenen Reaktionsgasen durch Gegenstromabsorption mit einem hochsiedenden hydrophoben Lösemittel abgetrennt. Aus dem anfallenden acrylsäurehaltigen Gemisch wird die Roh-Acrylsäure destillativ abgetrennt. Absorption von Acrylsäure in hochsiedenden Lösemitteln ist z.B. auch in der DE-OS 2 241 714 und DE-OS 43 08 087 beschrieben.

DE-OS 2 241 714 beschreibt die Verwendung von Estern aliphatischer oder aromatischer Mono- oder Dicarbonsäuren, die einen Schmelzpunkt von unter 30°C und einen Siedepunkt bei Normaldruck oberhalb von 160°C aufweisen.

DE-OS 43 08 087 empfiehlt für die Abtrennung von Acrylsäure aus Reaktionsgasen der katalytischen Oxidation durch Gegenstromabsorption die Verwendung eines hochsiedenden Gemisches aus 0,1 bis 25 Gew.-% ortho-Dimethylphthalat bezogen auf ein Gemisch, bestehend aus 70 bis 75 Gew.-% Diphenylether und 25 bis 30 Gew.-% Diphenyl.

Weit verbreitet ist auch die Absorption des Reaktionsgases in Wasser oder wässriger Acrylsäurelösung als Absorptionsmittel.

Anschließend wird die Roh-Acrylsäure durch destillative Abtrennung vom Absorptionsmittel erhalten.

Dabei kann die absorbierte Acrylsäure nach der Absorption oder vor der Destillation noch einem Desorptions- oder Strippprozess unterworfen werden, um den Gehalt an aldehydischen oder anderen carbonylhaltigen Nebenkomponenten zu verringern.

Es ist auch möglich, das Reaktionsgemisch aus der katalytischen Gasphasenoxidation zur Herstellung von Acrylsäure fraktionierend zu kondensieren, indem man es von unten in eine Kolonne mit trennwirksamen Einbauten führt und die kondensierbaren Komponenten durch Kühlung auskondensiert, wie es z.B. in der DE-A 197 40 253 beschrieben ist, oder nach einem dazu analogen Verfahren, in dem die Schwersiederfraktion über einen Seitenabzug abgeführt wird, wie in der deutschen Anmeldung mit dem Aktenzeichen 10053086.9 beschrieben.

Bevorzugt wird die erfindungsgemäß verwendbare Roh-Acrylsäure über eine fraktionierende Kondensation oder durch Absorption in Diphenylether-Diphenyl-Phthalsäureester-Gemischen erhalten.

Für das erfindungsgemäße Verfahren ist es unerheblich, nach welchem Verfahren die einsetzbare Roh-(Meth)acrylsäure erhalten worden ist.

Das erfindungsgemäße Verfahren besteht aus folgenden Stufen:

### 1. Vorbehandlung (optional)

Die nach einem beliebigen Verfahren hergestellte beim erfindungsgemäßen Verfahren eingesetzte Roh-Acrylsäure oder ein sonstiger essig- oder propionsäurehaltiger Acrylsäure-Strom kann beispielsweise folgende Komponenten enthalten:

| | |
|---|---|
| Acrylsäure | 90 - 99,9 Gew.-% |
| Essigsäure | 0,05 - 3 Gew.-% |
| Propionsäure | 0,01 - 1 Gew.-% |
| Diacrylsäure | 0,01 - 5 Gew.-% |
| Wasser | 0,05 - 10 Gew.-% |
| Furfural | 0,01 - 0,1 Gew.-% |
| Benzaldehyd | 0,01 - 0,05 Gew.-% |
| sonstige Aldehyde und andere carbonylhaltige | 0,01 - 0,3 Gew.-% |
| Inhibitoren | 0,01 - 0,1 Gew.-% |
| Maleinsäure(-anhydrid) | 0,001 -' 0,5 Gew.-% |

Bei Verwendung einer solchen Roh-Acrylsäure wird diese vorteilhaft vor Einsatz in der Veresterung in Gegenwart eines Amins, eines Hydrazins oder eines Hydrazinderivates, bevorzugt einem primären oder sekundären Amin oder Hydrazin(derivates) und besonders bevorzugt einem Hydrazin, in Mengen von 0,5 - 2 mol/mol carbonylhaltigen Verunreinigungen, bevorzugt 1 - 2 und besonders bevorzugt 1 - 1,5 mol/mol und einer Temperatur von 20 - 40°C für 0,1 - 10 Stunden, bevorzugt 0,5 - 7 und besonders bevorzugt 1 bis 5 Stunden behandelt.

Bevorzugt werden Aminophenole, Aminoguanidin und dessen Salze, wie z.B. Aminoguanidinhydrogencarbonat, Carbonsäurehydrazide, wie z.B. Adipinsäuredihydrazid, Anilin, Monoethanolamin, Diethanolamin, Hydrazin, Hydrazinhydrat, Phenylhydrazin, 4-Nitrophenylhydrazin oder 2,4-Dinitrophenylhydrazin verwendet, besonders'bevorzugt Hydrazinhydrat.

Die Umsetzung wird vorzugsweise in Gegenwart von 300 - 3000 ppm Phenothiazin als Stabilisator, oder einer gleichwirkenden Menge eines anderen geeigneten Stabilisators, durchgeführt.

Um zusätzliche Apparate zu vermeiden kann die Reaktion z.B. in einem Lagertank oder einem Vorlage- oder Zwischenbehälter durchgeführt werden, der bevorzugt mit einer Umwälzung, Rührung oder einem Umpumpkreislauf versehen ist.

Alternativ kann die Vorbehandlung auch in einem Rohrreaktor durchgeführt werden, der gegebenenfalls entweder begleitbeheizt ist, z.B. über einen Doppelmantel, oder der nach Erwärmung im Eingangsbereich, z.B. über Wärmetauscher, thermisch isoliert ist.

Wird als Ausgangssäure eine bei der Reinigung von Roh-Acrylsäure bei der Rein-Acrylsäure-Herstellung anfallende aldehydfreie acrylsäurehaltige Fraktion mit einem Carbonylgehalt unter 50 ppm, bevorzugt unter 10 ppm eingesetzt, so kann die beschriebene Vorbehandlung entfallen.

Selbstverständlich kann für das erfindungsgemäße Verfahren auch eine Rein-Acrylsäure eingesetzt werden, wobei in diesem Fall in der Regel keine Vorbehandlung vorgenommen wird.

Solche Rein-Acrylsäure kann beispielsweise folgende Zusammensetzung haben:

| | |
|---|---|
| Acrylsäure | 99,7 - 99,9 Gew.-% |
| Essigsäure | 50 - 1500 Gew.-ppm |
| Propionsäure | 10 - 500 Gew.-ppm |
| Diacrylsäure | 10 - 1000 Gew.-ppm |
| Wasser | 50 - 1000 Gew.-ppm |
| Aldehyde und andere Carbonylhaltige | 1 - 50 Gew.-ppm |
| Inhibitoren | 100 - 300 Gew.-ppm |
| Maleinsäure(-anhydrid) | 1 - 20 Gew.-ppm |

### 2. Veresterung

Das acrylsäurehaltige Gemisch, das gegebenenfalls aus der Vorbehandlung (Stufe 1) stammt, wird in Gegenwart mindestens eines sauren Katalysators mit dem Alkohol in einer Reaktionszone b) zur Reaktion gebracht.

Geeignete saure Katalysatoren sind Schwefelsäure, para-Toluolsulfonsäure, Benzolsulfonsäure, Dodecylbenzolsulfonsäure, Methansulfonsäure oder Gemische davon, denkbar sind auch saure Ionenaustauscher.

Bevorzugt werden Schwefelsäure, para-Toluolsulfonsäure und Methansulfonsäure verwendet, besonders bevorzugt Schwefelsäure.

Die Katalysatorkonzentration bezogen auf das Reaktionsgemisch beträgt beispielsweise 1 bis 20, bevorzugt 5 bis 15 Gew.-%.

Für die Reaktion geeignete Alkohole sind solche, die 1 bis 8 Kohlenstoffatome aufweisen, bevorzugt solche mit 1 bis 4 und besonders bevorzugt solche mit 1 bis 3 Kohlenstoffatomen.

Bevorzugt werden Methanol, Ethanol, n-Propanol, *iso*-Propanol, n-Butanol, *iso*-Butanol und 2-Ethylhexanol verwendet, besonders bevorzugt Methanol und Ethanol.

Die Zufuhr des Alkohols kann dabei flüssig oder/und gasförmig erfolgen.

Die Veresterung findet in mindestens einem beheizbaren Reaktor b1) statt, wobei durch geeignete Maßnahmen für eine gute Durchmischung gesorgt wird. Falls mehrere Reaktoren eingesetzt werden, z.B. zwei bis vier, so können diese in einer Kaskade angeordnet sein.

Vorzugsweise findet die Reaktion in einem Reaktor statt.

Der Reaktor b1) ist mit mindestens einer Destillationseinheit verbunden, die bevorzugt 30 - 50 theoretische Böden aufweist. Bevorzugt ist die Destillationseinheit b2) auf den Reaktor b1) aufgesetzt.

Es können auch mehrere Reaktoren mit einer Destillationseinheit verbunden sein. Der Rückfluß aus der Destillationseinheit wird dann bevorzugt in den ersten Reaktor zurückgeführt.

Die Destillationseinheit ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glokkenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak oder Geflechten bevorzugt.

Der Kondensator, so vorhanden, ist von herkömmlicher Bauart.

Eine bevorzugte Ausführungsform besteht darin, daß der Sumpfbereich und der Verdampfer einer Destillationseinheit als Reaktor b1) verwendet wird.

Wird der Alkohol gasförmig zugeführt (s.u.), so ist die bevorzugte Dosierstelle unterhalb der trennwirksamen Einbauten der Destillationseinheit b2) oder im Umlaufkreislauf.

Das Reaktionsgemisch wird mit einem geeigneten Stabilisator, z.B. Phenothiazin (0,05 - 0,5 % bzgl. Reaktionsgemisch) gegen unerwünschte Polymerisation stabilisiert, wobei die Zufuhr des Stabilisators vorzugsweise mit der Acrylsäure erfolgt.

Die Reaktion findet bei 120 - 150°C und Umgebungsdruck statt, es kann auch höherer oder verminderter Druck verwendet werden, bevorzugt ist Umgebungsdruck.

Die Reaktionszeit beträgt in der Regel 0,5 - 10 Stunden, vozugsweise 1 - 6 Stunden.

Die Ausgangsstoffe Acrylsäure und Alkohol werden in der Regel in der Stöchiometrie 1: 0,7 - 3,0, bevorzugt 1: 0,9 - 2,5, besonders bevorzugt 1: 1,0 - 2,0 und insbesondere 1 : 1,0 - 1,5 zudosiert.

Der bei der Veresterung entstehende Zielester, Leichtsieder, die Michael-Additionsprodukte, unter diesen bevorzugt der Alkoxypropionsäureester, und das gebildete Reaktionswasser werden über die mit dem Veresterungsreaktor b1) verbundene Kolonne b2) als Kopfprodukt abgetrennt (Kopftemperatur 70 - 90°C, Kopfdruck 1 bar). Das kondensierte Kopfprodukt (Temperatur in der Regel 20 bis 40°C) wird mit einem Inhibitor stabilisiert und besteht im wesentlichen aus Zielester, nicht umgesetztem Alkohol, Wasser, Essigsäureester, Michael-Additionsprodukt, wie Alkoxypropionsäureester, und verschiedenen Nebenkomponenten. Der Acrylsäuregehalt im Kopfprodukt beträgt in der Regel nicht mehr als 0,1 %, bevorzugt nicht mehr als 0,01 %.

Als Inhibitoren können Alkylphenole, beispielsweise o-, m- oder p-Kresol (Methylphenol), 2-tert.-Butyl-4-methylphenol, 6-tert.-Butyl-2,4-dimethyl-phenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol), Hydroxyphenole, beispielsweise Hydrochinon, 2-Methylhydrochinon, 2,5-Di-tert.-Butylhydrochinon, Brenzcatechin (1,2-Dihydroxybenzol) oder Benzochinon, Aminophenole, wie z.B. para-Aminophenol, Nitrosophenole, wie z.B. para-Nitrosophenol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, Tocopherole, wie z.B. α-Tocopherol sowie 2,3-Dihydro-2,2-dimethyl-7-hydroxybenzofuran (2,2-Dimethyl-7-hydroxycumaran), N-Oxyle, wie 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, aromatische Amine oder Phenylendiamine, wie z.B. N,N-Diphenylamin, N-Nitroso-diphenylamin, N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, phosphorhaltige Verbindungen, wie z.B. Triphenylphosphin, Triphenylphosphit, Hypophosphorige Säure oder Triethylphosphit, schwefelhaltige Verbindungen, wie z.B. Diphenylsulfid oder Phenothiazin, gegebenenfalls in Kombination mit Metallsalzen, wie beispielsweise die Chloride, Dithiocarbamate, Sulfate, Salicylate oder Acetate von Kupfer, Mangan, Cer, Nickel oder Chrom verwendet werden.

Bevorzugt sind an dieser Stelle wasserlösliche Stabilisatoren, besonders bevorzugt N,N'-Dialkyl-p-phenylendiamine, wie z.B. N,N'-Di-*iso*-butyl-p-phenylendiamin oder N,N'-Di-iso-propyl-p-phenylendiamin, und N-Oxyle, insbesondere para-Phenylendiamin oder 4-Hydroxy-2,2,6,6-tetramethyl-piperidin-N-oxyl.

Der Stabilisator wird in Mengen von 10 - 1000 ppm, bevorzugt 50 bis 500 ppm, bzgl. des Destillats eingesetzt.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich einer Kolonne und/oder in einen Umlaufverdampfer eindosiert.

Es kann alternativ auch auf eine Kondensation verzichtet werden, in diesem Fall wird das Destillat im wesentlichen gasförmig in die nachfolgende Wäsche (Stufe 3) geführt.

Als Rücklauf wird das in Stufe 3 anfallende weitgehend alkoholfreie acrylsäureesterhaltige Gemisch c1) auf den obersten Kolonnenboden aufgebracht. Bevorzugt werden 20 - 80 Gew.-%, bezogen auf das Reaktionsgemisch, besonders bevorzugt 30 - 60 und ganz besonders bevorzugt 40 - 60 Gew.-% als Rücklauf aufgebracht.

Ein Teil des Veresterungssumpfes, bevorzugt 0,1 - 1 % bezüglich Zielester, wird als b3) kontinuierlich abgetrennt, um Hochsieder auszuschleusen und kann entweder entsorgt, z.B. verbrannt, oder einer Hochsiederaufarbeitung (Stufe 8) zugeführt werden.

Die Veresterung wird so betrieben, daß der Sumpf max. 10 % Zielester und max. 15 % Acrylsäure enthält.

Eine weitere Verfahrensvariante besteht darin, daß man die Veresterung in einem beheizbaren Vorreaktor, gegebenenfalls unter Druck, durchführt und das so erhältliche, flüssige Reaktionsgemisch einer Destillationseinheit, bestehend aus Kolonne, Umlaufverdampfer und Kondensator, zuführt. Das Reaktionsgemisch wird aufgetrennt wie oben beschrieben. Das katalysatorhaltige Sumpfprodukt wird ganz oder teilweise in den Reaktor zurückgeführt.

### 3. Wäsche

Das in Stufe 2 anfallende Destillat beziehungsweise Kondensat, das im wesentlichen aus Acrylsäureester (75 - 90 %), Alkohol (1 - 10 %), Wasser (7 - 13 %), Michael-Additionsprodukt, besonders Alkoxypropionsäurealkylester, (0,5 - 2,5 %), Essigsäureester (0,05 - 1 %) und diversen Leichtsiedern, z.B. Propionsäureester, Aldehyde und Ether, (0,5 - 3 %) besteht, wird, gegebenenfalls zusätzlich mit weiterem Stabilisator versetzt, einer Wäsche mit einer Waschflüssigkeit unterworfen.

Die Menge der Waschflüssigkeit beträgt 10 - 200 Gew.-% bezogen auf das Destillat/Kondensat, bevorzugt 40 - 150 Gew.-% und besonders bevorzugt 50 - 100 Gew.-%.

Bei der Waschflüssigkeit handelt es sich beispielsweise um Wasser, dem gegebenenfalls noch basische Verbindungen, beispielsweise Natriumhydroxid, Kaliumhydroxid, Natriumhydrogencarbonat, Natriumcarbonat, Kaliumhydrogencarbonat oder Kaliumcarbonat zugesetzt sein können, bevorzugt wird Wasser verwendet.

Als Waschflüssigkeit kann Trinkwasser, Kondensat oder deionisiertes Wasser, gegebenenfalls mit den obigen Zusätzen versehen, verwendet werden.

Eine weitere Ausführungsform besteht darin, die im Prozess anfallenden wässrigen Phasen zu verwenden oder mitzuverwenden, z.B. aus Phasenscheidern, beispielsweise solche aus den Stufen 4 oder 5, oder Wasser aus Vakuumaggregaten, z.B. Wasserringpumpen, insbesondere die wässrige Fraktion d2) aus der Alkoholrückgewinnung (Stufe 4).

Erfindungsgemäß wesentlich ist, daß durch die Wäsche unumgesetzter Alkohol aus der Veresterung sowie andere in der Waschflüssigkeit lösliche Nebenprodukte entfernt werden.

Verfahrenstechnisch können für eine Wäsche im erfindungsgemäßen Verfahren prinzipiell alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt mehrstufige Extraktionen, sowie solche in Gleich- oder Gegenstromfahrweise, bevorzugt Gegenstromfahrweise, sein.

Vorzugsweise werden Siebboden-, Packungs- beziehungsweise Füllkörperkolonnen, Rührbehälter oder Mixer-Settler-Apparate, sowie Kolonnen mit rotierenden Einbauten oder pulsierte Kolonnen eingesetzt.

Besonders bevorzugt wird eine Kolonne mit 70 bis 150 theoretischen Böden verwendet. Als Kolonneneinbauten kommen prinzipiell alle gängigen Einbauten in Betracht, beispielsweise Böden, Pakkungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt.

Das Destillat/Kondensat aus Stufe 2 wird bevorzugt am unteren Ende der Kolonne, die Waschflüssigkeit bevorzugt am Kopf zugeführt.

Die am Kolonnenkopf austretende organische Phase wird in ein an sich bekanntes Trenngefäß geleitet, um restliches Wasser als Phase c2) abzutrennen, und mit einem Inhibitor stabilisiert.
Die weitgehend alkoholfreie Esterphase c1), die in der Regel einen Alkoholgehalt von nicht mehr als 0,1 Gew.-%, bevorzugt nicht mehr als 0,02 % aufweist, wird teilweise der Destillationseinheit b2) in Stufe 2 als Rücklauf zugeführt und teilweise einer weiteren destillativen Aufreinigung des Acrylsäureesters (Stufe 5) im Verhältnis von 30:70 - 70:30, bevorzugt im Verhältnis von 40:60 - 60:40 zugeführt.

Die organische Phase c1) wird dabei erfindungsgemäß ohne weitere Wäschen oder Neutralisationen destillativ aufgearbeitet.

Die wässrige Phase c2) aus dem Phasenscheider wird bevorzugt vollständig in die Alkoholrückgewinnung (Stufe 4) geführt.

Als Inhibitoren können auch hier die o.g. Alkylphenole, Alkoxyphenole, Hydroxyphenole, Aminophenole, Tocopherole, N-Oxyle, aromatischen Amine oder Phenylendiamine, Hydroxylamine, phosphorhaltigen Verbindungen oder schwefelhaltigen Verbindungen gegebenenfalls in Kombination mit Metallsalzen verwendet werden.

Bevorzugt sind an dieser Stelle wasserlösliche Stabilisatoren, besonders bevorzugt N,N'-Dialkyl-para-phenylendiamine und N-Oxyle, insbesondere N,N'-Di-*iso*-butyl-para-phenylendiamin oder 4-Hydroxy-2,2,6,6-tetramethyl-piperidin-N-oxyl.

Der Stabilisator wird in Mengen von 10 - 1000 ppm, bevorzugt 50 bis 500 ppm, bezogen auf die am Kolonnenkopf austretende organische Phase eingesetzt.

Die am unteren Ende der Kolonne austretende Wasserphase c3), die im allgemeinen 5 - 10 Gew.-% Alkohol, 3 - 7 Gew.-% Acrylester, 0,1 - 1 Gew.-% Michael-Additionsprodukt wie Alkoxypropionsäurealkylester und 0,1 - 1 Gew.-% Leichtsieder enthält, wird in der Stufe 4 (Alkoholrückgewinnung) ganz oder teilweise aufgearbeitet. Ein Teil dieser Wasserphase c3) kann auch wieder als Waschflüssigkeit in die Wäsche rückgeführt werden.

Eine weitere Möglichkeit besteht darin, daß das Destillat aus Stufe 2 nicht kondensiert wird, sondern gasförmig der Stufe 3 zugeführt und mit der Waschflüssigkeit gequencht wird.

Um die Abwassermenge zu verringern kann es sinnvoll sein, die Wäsche (Stufe 3) ganz oder teilweise zu umgehen, beispielsweise indem man das am Kopf der Kolonne b2) anfallende Destillat/Kondensat ganz oder teilweise direkt einer destillativen Aufarbeitung, beispielsweise der Leichtsiederabtrennung (Stufe 5) zuführt, wobei gegebenenfalls ein Phasentrenngefäß zur Abtrennung des bei der Reaktion entstehenden Wassers vorgeschaltet sein kann. Das bei der destillativen Aufarbeitung anfallende Sumpfprodukt, z.B. e3), kann gegebenenfalls teilweise als Rücklauf für die Kolonne b2) verwendet werden.

### 4. Alkoholrückgewinnung

Die in Stufe 3 anfallenden Wasserphasen c2) und c3) aus Kolonne und Trennbehälter werden gegebenenfalls gemeinsam mit der in Stufe 5 und den bei der Vakuumerzeugung (Wasserringpumpen) anfallenden Wasserphasen einer Wertproduktrückgewinnung von Alkohol, Acrylester und Michael-Additionsprodukten zugeführt.

Die Rückgewinnungseinheit besteht bevorzugt aus einer Destillationskolonne d) mit Verdampfer, Kondensator jeweils üblicher Bauart und einem Seitenabzug.

Die Kolonne weist bevorzugt 30-70 theoretische Böden auf, z.B. Böden, Packungen und/oder Schüttungen. Von den Böden sind Glokkenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak oder Geflechten bevorzugt.

Der Zulauf der vereinigten Wasserphasen erfolgt am oberen Ende der unteren Kolonnenhälfte, wobei der Zulauf vorzugsweise durch Wärmeaustausch mit dem Ablauf der Alkoholrückgewinnungskolonne auf 40 - 90°C, bevorzugt 60 - 90 °C erhitzt wird.

Die Sumpftemperatur beträgt 100 - 110°C, die Kopftemperatur 60 - 80°C bei leicht vermindertem oder Atmosphärendruck, bevorzugt bei Atmosphärendruck.

Die am Kolonnenkopf austretenden Brüden werden kondensiert, mit einem Inhibitor stabilisiert und teilweise wieder der Kolonne d) als Rücklauf zugeführt. Der übrige Teil des Kondensats d1) wird direkt der Veresterung (Stufe 2) zugeführt. Dabei kann der Zulauf zur Stufe 2, wenn er flüssig ist etwa in der Mitte der Kolonne b2), in den Reaktor b1) oder in dessen Zulauf, oder, wenn er gasförmig ist, unterhalb der trennwirksamen Einbauten oder in den Umlaufkreislauf dosiert werden.

Die Brüden bestehen im wesentlichen aus Alkohol (40 - 70 und Acrylsäureester (30 - 50 ö).

Als Inhibitoren können auch hier die o.g. Alkylphenole, Hydroxyphenole, Aminophenole, Tocopherole, N-Oxyle, aromatischen Amine oder Phenylendiamine, Hydroxylamine, phosphorhaltigen Verbindungen oder schwefelhaltigen Verbindungen gegebenenfalls in Kombination mit Metallsalzen verwendet werden.

Bevorzugt sind an dieser Stelle wasserlösliche Stabilisatoren, besonders bevorzugt N,N'-Dialkyl-para-phenylendiamine und N-Oxyle, insbesondere N,N'-Di-iso-butyl-para-phenylendiamin oder 4-Hydroxy-2,2,6,6-tetramethyl-piperidin-N-oxyl.

Der Stabilisator wird in Mengen von 10 - 500 ppm, bevorzugt 50 bis 300 ppm, bezogen auf das Destillat eingesetzt.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich der Kolonne und/oder in einen Umlaufverdampfer eindosiert.

Es kann auch nur teilweise kondensiert werden, bevorzugt der Teil, der für den Rücklauf erforderlich ist, und die gasförmigen Brüden direkt in die Veresterung geleitet werden.

Vorzugsweise wird im unteren Teil der oberen Kolonnenhälfte eine Mittelsiederfraktion d3) in gasförmiger oder flüssiger Form über einen Seitenabzug aus der Kolonne ausgeschleust, die hauptsächlich Michael-Additionsprodukte, besonders Alkoxypropionsäurealkylester, (5 - 10 Gew.-%), Alkohol (40 - 60 Gew.-%) und Wasser enthält und wieder dem Reaktor b1) der Veresterung (Stufe 2) zugeführt wird. Dort werden die Michael-Additionsprodukte, besonders Alkoxypropionsäurealkylester, zumindest teilweise in Alkohol und Acrylester zurückgespalten und der so freigesetzte Alkohol mit der vorhanden Acrylsäure verestert.

Die im Sumpf der Rückgewinnungskolonne d) anfallende Wasserphase d2) wird, bevorzugt durch Wärmeübertragung auf den Kolonnenzulauf von d) , in einem Wärmeaustauscher abgekühlt, zumindest teilweise in die Wäsche (Stufe 3) zurückgeführt und dort als Waschflüssigkeit verwendet und teilweise ausgeschleust, bevorzugt zu 1 - 50 %, besonders bevorzugt zu 5 - 40 % und insbesondere zu 10 bis 30 %. Der ausgeschleuste Teil kann in an sich bekannter Weise entsorgt werden, z.B. über eine Kläranlage.

Eine weitere bevorzugte Ausführungsform besteht darin, daß man den zur Veresterung benötigten Frischalkohol am Kopf der Kolonne d) als Rücklauf aufbringt und die Brüden direkt, bevorzugt gasförmig der Reaktionszone b) der Veresterung (Stufe 2) zuführt. Der Zulauf erfolgt dabei wie oben beschrieben.

### 5. Leichtsiederabtrennung

Die Leichtsiederabtrennung wird insbesondere dann durchlaufen, wenn Rohacrylsäure als Einsatzstoff verwendet wird und/oder ein besonders leichtsiederarmer Acrylsäureester hergestellt werden soll.

Ein Teil des in Stufe 3 anfallenden weitgehend alkoholfreien Acrylsäureestergemisches (organische Phase c1) des Phasenscheiders aus Stufe 3 wird in einer Destillationseinheit e), bestehend aus einer Destillationskolonne, einem Verdampfer und einem Kondensator mit Phasentrenngefäß jeweils üblicher Bauart, in eine Leichtsiederfraktion und ein Sumpfprodukt e3), das den Zielester enthält, aufgetrennt.

Die Kolonne weist bevorzugt 20 - 60 theoretische Böden und die in Stufe 4 beschriebenen Einbauten auf.

Der Zulauf befindet sich bevorzugt oberhalb der Kolonnenmitte.

Die Sumpf temperatur beträgt zwischen 80 und 100 °C, die Kolonne wird bei Normaldruck oder leicht vermindertem Druck betrieben, beispielsweise 500 mbar - Normaldruck, bevorzugt 700 mbar - Normaldruck und besonders bevorzugt 800 mbar - Normaldruck.

Die kondensierte Leichtsiederfraktion zerfällt in eine Wasserphase e2), die in die Alkoholrückgewinnungsstufe (Stufe 4) und/ oder in die Wäsche (Stufe 3) zurückgeführt wird, und in eine organische Phase e1), die hauptsächlich Essigsäurealkylester, Ether und Alkylacrylat enthält.

Die organische Phase e1) wird mit einem Stabilisator versetzt und teilweise als Rücklauf auf den obersten Kolonnenboden der Kolonne e) aufgebracht (Rücklaufverhältnis 20 - 40) und kann teilweise einer weiteren Destillationseinheit (Stufe 6) zugeführt werden.

Als Inhibitoren können hier die o.g. Alkylphenole, Alkoxyphenole, Hydroxyphenole, Aminophenole, Tocopherole, N-Oxyle, aromatischen Amine oder N,N'-Dialkyl-para-phenylendiamine, Hydroxylamine, phosphorhaltigen Verbindungen oder schwefelhaltigen Verbindungen gegebenenfalls in Kombination mit Metallsalzen verwendet werden.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich der Kolonne und/oder in einen Umlaufverdampfer eindosiert.

Bevorzugt sind an dieser Stelle Gemische aus Phenothiazin und p-Nitrosophenol und/oder Nitrosodiethylanilin und/oder Tetramethylpiperidin-1-oxyle und/oder N,N'-Di-*iso*-butyl-para-phenylendiamin eingesetzt.

Der Kondensator der Stufe 5 wird vorzugsweise mit einer Lösung eines teilweise wasserlöslichen Inhibitors im Zielester beaufschlagt, bevorzugt durch Einsprühen, um eine Polymerisationsbildung an den Kühlflächen zu verhindern.

Bevorzugt sind dafür p-Phenylendiamine und N-Oxyle, besonders bevorzugt para-Phenylendiamin oder 4-Hydroxy-2,2,6,6-tetramethylpiperidin-N-oxyl.

### 6. Leichtsiederaufarbeitung

Die Leichtsiederaufarbeitung wird insbesondere dann durchlaufen, wenn Rohacrylsäure als Einsatzstoff verwendet wird und/oder ein besonders leichtsiederarmer Acrylsäureester hergestellt werden soll.

Der nicht als Rücklauf verwendete Teil der organischen Phase e1) der Leichtsiederfraktion aus Stufe 5 kann in einer weiteren Destillationseinheit f) mit Kondensator und Phasentrenngefäß üblicher Bauart in eine Leichtsiederfraktion f1), hauptsächlich Alkylacetat und Ether enthaltend, und in ein im wesentlichen aus Alkylacrylat bestehendes Sumpfprodukt f2) aufgetrennt werden. Das Kondensat f1) wird teilweise ausgeschleust und z.B. thermisch verwertet oder verseift und teilweise, nach Stabilisierung analog Stufe 5, als Rücklauf mit einem Rücklaufverhältnis von beispielsweise 20 - 40:1 in die Destillationskolonne f) zurückgeführt.

Der ausgeschleuste Anteil kann auch alkalisch, beispielsweise mit Natronlauge, verseift werden, um den im Acetat enthaltenen Alkohol zurückzugewinnen. Der Verseifungsaustrag kann dann beispielsweise der Alkoholrückgewinnung d) zugeführt werden.

Der im wesentlichen Acrylsäureester enthaltende Sumpf f2) der Kolonne wird in Stufe 5 zurückgeführt.

Eine weitere Möglichkeit besteht darin, den Sumpf f2) als Rücklauf in der Destillationseinheit b2) der Stufe 2 zu verwenden.

Die Sumpftemperatur beträgt 60 bis 80°C, der Druck ist bevorzugt Normaldruck oder leicht verminderter Druck, beispielsweise 500 mbar - Normaldruck, bevorzugt 700 mbar - Normaldruck und besonders bevorzugt 800 mbar - Normaldruck.

Eine bevorzugte Ausführungsform besteht darin, daß eine Packungskolonne mit bevorzugt 10 - 30 theoretischen Böden verwendet wird.

### 7. Reindestillation

Aus dem in Stufe 5 anfallenden Sumpfprodukt e3), das eine Reinheit von in der Regel mindestens 98 %, bevorzugt mindestens 99 % aufweist, beziehungsweise, wenn die Stufen 5 und 6 nicht durchlaufen wurden, aus der organischen Phase c1) wird in einer weiteren Destillationsstufe g) in einer Destillationskolonne mit bevorzugt 5 - 20 theoretischen Böden und Verdampfer und Kondensator üblicher Bauart bei einer Sumpftemperatur zwischen 40 und 80°C und einem Kopfdruck von 0,1 - 0,7 bar, bevorzugt 0,2 - 0,6 bar, der Zielester als Kopfprodukt g1) isoliert. Der Kondensator wird dabei mit einer Lösung eines Lagerstabilisators im Zielester beaufschlagt, bevorzugt wird die Stabilisatorlösung eingesprüht.

Als Kolonneneinbauten für die Kolonne kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt.

Als Inhibitoren können hier die o.g. Alkylphenole, Alkoxyphenole, Hydroxyphenole, Aminophenole, Tocopherole, N-Oxyle, aromatischen Amine oder N,N'-Dialkyl-para-phenylendiamine, gegebenenfalls in Kombination mit Metallsalzen verwendet werden.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich der Kolonne und/oder in einen Umlaufverdampfer eindosiert.

Bevorzugt wird Hydrochinonmonomethylether als Lagerstabilisator verwendet.

Die Menge wird so eingestellt, daß der Lagerstabilisatorgehalt im Kondensat 10 - 20 ppm beträgt.

Ein Teil des Zielesters wird mit dem Inhibitorgemisch aus Stufe 5 (s.o.), bevorzugt mit 50 - 100 ppm versetzt, und als Rücklauf der Kolonne zugeführt (Rücklaufverhältnis 0,1 - 1 : 1, bevorzugt 0,1 - 0,7 : 1, besonders bevorzugt 0,1 - 0,5 : 1). Der Sumpf der Kolonne g2), der hauptsächlich Alkylacrylat und Michael-Additionsprodukt, besonders Alkoxypropionsäurealkylester enthält, wird vorzugsweise dem Reaktor b1) zugeführt, wo unter den Veresterungsbedingungen die Michael-Additionsprodukte zumindest teilweise in Alkohol und Acrylat zurückgespalten wird.

Die nach dem erfindungsgemäßen Verfahren erhältlichen Acrylsäureester haben eine Reinheit von mindestens 99 %, bevorzugt 99,5 %, besonders bevorzugt von mindestens 99,8 % und insbesondere von mindestens 99,9% und haben einen Gehalt an Alkylpropionat von nicht mehr als 1000 ppm, bevorzugt nicht mehr als 500 ppm, einen Gehalt an Alkylacetat von nicht mehr als 100 ppm, bevorzugt nicht mehr als 50 ppm und einen Gehalt an Acrylsäure von nicht mehr als 100 ppm, bevorzugt nicht mehr als 50 ppm.

### 8. Hochsiederaufarbeitung (fakultativ)

Der Strom b3) aus der Veresterung (Stufe 2) kann optional zumindest teilweise einer Hochsiederspaltung unterworfen werden.

Dazu wird der Strom b3) oder gegebenenfalls auch die Michaeladditionsprodukte enthaltenden Ströme d3) und/oder g2) einem Reaktor oder einem gegebenenfalls im Kreislauf betriebenen Destillationsapparat zugeführt und dort thermisch und/oder katalytisch behandelt.

Die Temperatur in der Rückspaltung beträgt im allgemeinen 110 bis 220°C, bevorzugt 120 bis 200°C und besonders bevorzugt 130 bis 180°C.

Die Abtrennung des restlichen Acrylsäureesters und der dabei entstehenden Spaltprodukte kann durch das Durchleiten eines unter den Reaktionsbedingungen im wesentlichen inerten Gasstromes (Strippen), wie z.B. Stickstoff, Wasserdampf, oder vorzugsweise eines sauerstoffhaltigen Gases, wie z.B. Luft, unterstützt werden.

Der verbleibende Rückstand kann beispielsweise teilweise ausgeschleust, destilliert oder nochmals der Rückspaltung unterworfen oder zur Gewinnung von Schwefelsäure eingesetzt werden.

Der aus der Rückspaltung erhaltene gasförmige Strom, der im wesentlichen Acrylsäureester, Alkohol, Acrylsäure sowie gegebenenfalls Ether und auch Stabilisator enthalten kann, kann, gegebenenfalls nach Kondensation und/oder weiterer Abkühlung, bevorzugt in den Veresterungsreaktor b1) oder unterhalb der trennwirksamen Einbauten von b2) oder in den Umlaufkreislauf der Reaktionszone b) geführt werden.

Anstelle dieser Hochsiederaufarbeitung kann der Strom b3) auch vorteilhaft zur Gewinnung von Schwefelsäure eingesetzt werden.

Dazu wird ein schwefelhaltiger Strom in an sich bekannter Weise in einen schwefeloxidhaltigen Strom überführt und z.B. in einer Kontaktanlage zu Schwefelsäure umgesetzt.

Die Vorteile des erfindungsgemäßen Verfahrens sind
- Verwendbarkeit einer rohen Acrylsäure, d.h. einer Acrylsäure, die noch Essigsäure, Propionsäure, Aldehyde und Maleinsäureanhydrid enthalten kann
- nur geringe Belegung (Fouling) des Reaktors und der Verdampfer
- Verminderung der Abwassermengen durch Rückführung
- Vermeidung von Wertproduktverlusten durch Aufarbeitung der anfallenden Abwässer und der Leichtsiederfraktion und dadurch
- geringe Umweltbelastung
- lange Laufzeiten der Destillationseinheiten
- hohe Ausbeute (mindestens 98% bzgl. (Meth)acrylsäure)
- Zielester mit sehr hoher Reinheit (>99,9 %, aldehydfrei)
- kein Auftreten von festem Polymerisat, z.B. in Verdampfern

Die in dieser Schrift angegebenen Teile-, Prozent- und ppm-Angaben beziehen sich, wenn nicht anders angegeben, auf Gewichtsteile, Gew.-% und Gew.-ppm.

### Beispiel 1

Es wurde Roh-Acrylsäure folgender Zusammensetzung eingesetzt:

| | |
|---|---|
| 99,6 % | Acrylsäure |
| 0,18 % | Essigsäure |
| 0,03 % | Propionsäure |
| 0,05 % | Furfurale |
| 0,01 % | Benzaldehyd |
| 0,06 % | Wasser |
| 0,01 % | Maleinsäureanhydrid |
| 0,05 % | Phenothiazin |

Die Roh-Acrylsäure wurde vor der Veresterung bei 23 °C mit Hydrai zin-Hydrat (1,5 Mol/Mol Aldehyd) behandelt (Verweilzeit 1 Stunde).

In den Sumpf einer Kolonne mit 40 Dual-Flow-Böden und außenliegendem Umlaufverdampfer wurden stündlich 5360 Teile behandelte Roh-Acrylsäure, 676 Teile Sumpfprodukt der Reinkolonne, 400 Teile Michael-Additionsprodukt enthaltende Fraktion der Methanol-Rückgewinnung und 20 Teile Schwefelsäure (96 %-ig) zugeführt. Unterhalb des 1. Bodens der Kolonne wurden stündlich 4302 Teile des in der Methanol-Rückgewinnung anfallenden Kopfproduktes gasförmig zugeführt. Aus dem Sumpf des Reaktors wurden kontinuierlich 70 Teile ausgeschleust, hauptsächlich Katalysator, hochsiedende Nebenprodukte und Inhibitoren enthaltend. Auf den obersten Kolonnenboden wurden 7000 Teile gewaschener Rohester zugeführt. Die Reaktionstemperatur betrug 130°C.

Am Kopf der Kolonne wurden 17622 Teile/h eines Gemisches, das im wesentlichen aus Methylacrylat (82,1 %), Wasser (9,5 %), Methanol (6,5 %), Leichtsieder (1,0 %) und Methoxypropionsäuremethylester (0,9 %) bestand, ausgeschleust und kondensiert. Der Kondensator wurde mit einer Stabilisatorlösung (160 Teile/h), bestehend aus einer Lösung von 0,4 % N,N'-Di-*iso*-butyl-p-phenylendiamin in Methylacrylat, beaufschlagt.

Das Rohestergemisch wurde in einer Waschkolonne (120 Böden) mit 10000 Teile/h Abwasser der Methanol-Rückgewinnung behandelt, wobei noch die Wasserphase des Phasentrenngefäßes der Leichtsiederdestillation (179 Teile/h) und das Abwasser der Vakuumpumpen (580 Teile/h) zugegeben wurden. Die gewaschene organische Phase wurde mit 160 Teile/h Stabilisatorlösung (s.o.) versetzt und teilweise (7000 Teile/h) als Rücklauf der Reaktorkolonne und teilweise (7242 Teile/h) gemeinsam mit dem Sumpfprodukt der Leichtsiederaufarbeitung (86 Teile /h) der Leichtsiederabtrennung zugeführt.

Das Abwasser der Waschkolonne wurde auf 40°C erwärmt und auf den 25. Boden der Methanol-Rückgewinnungs-Kolonne (60 Dual-Flow-Böden, außenliegender Umlaufverdampfer, 105 °C Sumpftemperatur) zugeführt. Am Kopf der Kolonne wurden 2400 Teile Methanol (99,9 Gew.-%, Frischzulauf) als Rücklauf und 100 Teile Stabilisatorlösung (s.o.) aufgebracht. Das anfallende Destillat, das hauptsächlich aus Methanol (77,8 %) und Methylacrylat (20,6 %) bestand, wurde gasförmig in die Veresterung zurückgeführt. Über einen Seitenabzug (40. Boden) wurden flüssig 400 Teile/h einer Michael-Additionsprodukt enthaltenden Fraktion ausgeschleust (6 % Methoxypropionsäuremethylester, 48 % Methanol und 56 % Wasser) und dem Veresterungsreaktor zudosiert.

Das methanolfreie Abwasser wurde teilweise in die Wäsche zurückgeführt (10000 Teile/h) und teilweise ausgeschleust (2100 Teile/h).

Die Leichtsiederabtrennung erfolgte in einer Kolonne mit 52 Dual-Flow-Böden (Zulauf auf 32. Boden) und Umlaufverdampfer bei einer Sumpftemperatur von 88°C. Die am Kopf der Kolonne ausgeschleuste Leichtsiederfraktion zerfiel in eine Wasserphase (179 Teile/h), die der Extraktionskolonne zugeführt wurde, und eine organische Phase, die teilweise als Rücklauf (2000 Teile/h) in die Kolonne zurückgeführt wurde und teilweise (60 Teile/h) der Leichtsiederaufarbeitung zugeführt wurde. Der Kondensator wurde mit 200 Teile/h Stabilisatorlösung bestehend aus 0,2 % Phenothiazin und 0,1 % N,N'-Di-iso-butyl-p-phenylendiamin in Acrylsäuremethylester beaufschlagt.

Das Sumpfprodukt (7796 Teile/h), hauptsächlich aus Methylacrylat (99 %) bestehend, wurde in der Reinkolonne in ein Kopfprodukt, dem Zielester (7335 Teile/h, 98,5 % Ausbeute bzgl. Acrylsäure), und ein Sumpfprodukt, das im wesentlichen aus 89 % Methylacrylat und 10,5 % Michael-Additionsprodukt bestand und wieder der Veresterung zugeführt wurde, aufgetrennt.

Die Reindestillation erfolgte in einer Kolonne mit 15 Dual-Flow-Böden und einem außenliegenden Umlaufverdampfer. Die Kopftemperatur betrug 43°C (0,3 bar), das Rücklaufverhältnis 0,3. In das Brüdenrohr wurden 160 Teile/h einer Lösung von 0,2 % Hydrochinonmonomethylether in Methylacrylat eingesprüht und die Kondensatorrohre mit 100 Teile/h dieser Lösung besprüht. Das Rücklaufverhältnis betrug 0,3, der Rücklauf wurde mit 120 Teilen Stabilisatorgemisch (s.o.) versetzt.

Die Leichtsiederaufarbeitung erfolgte in einer Packungskolonne (16 Trennstufen) bei einer Sumpftemperatur von 72°C bei Atmosphärendruck und einem Rücklaufverhältnis von 25. Das Sumpfprodukt, hauptsächlich Methylacrylat (94 %), wurde wieder der Leichtsiederabtrennung zugeführt, das nicht als Rücklauf verwendete Kopfprodukt (19 Teile/h) wurde ausgeschleust. Der Rücklauf der Kolonne wurde mit 50 Teilen/h der Stabilisatorlösung (s.o.) versetzt.

Das gewonnene Methylacrylat hatte entsprechend der gaschromatographischen Analyse (analog DIN 55 686) eine Reinheit von 99,96 % und enthielt als Nebenkomponenten noch 310 ppm Methylpropionat, 25 ppm Acrylsäure, 60 ppm Wasser, weniger als 10 ppm Methylacetat und weniger als 1 ppm Aldehyde.

Die Anlage konnte mindestens 30 Tage störungsfrei betrieben werden.

### Vergleichsbeispiel 1

Es wurde wie in Beispiel 1 verfahren, jedoch wurde die gleiche Roh-Acrylsäure ohne Vorbehandlung mit Hydrazin eingesetzt.

Nach 1 Woche mußte die Anlage wegen Belagbildung am Verdampfer abgestellt werden.

### Vergleichsbeispiel 2

Es wurde wie in Beispiel 1 verfahren, jedoch ohne die Leichtsiederabtrennung und -ausschleusung.

Die Reinheit des Produktes betrug 99,78 %, es waren 1700 ppm Methylacetat und 300 ppm Methylpropionat enthalten.

## Patentansprüche

1. Verfahren zur Herstellung von (Meth)acrylsäureestern durch Umsetzung von (Meth)acrylsäure mit einem Carbonylgehalt unter 50 ppm mit einem Alkohol in Gegenwart mindestens eines sauren Katalysators und mindestens eines Polymerisationsinhibitors in einer Reaktionszone b), bestehend aus mindestens einem Reaktor b1) mit mindestens einer angeschlossener Destillationseinheit b2), **dadurch gekennzeichnet, daß** man
- im Reaktor b1) die Temperatur auf 120 bis 150 °C und
- die gewünschte Stöchiometrie (Meth)acrylsäure: Alkohol einstellt,
- das Destillat der Destillationseinheit b2) mit einer Waschflüssigkeit behandelt (Waschschritt c)),
- den Austrag aus dem Waschschritt c) in mindestens eine organische c1) und mindestens zwei wässrige Phasen c2) und c3) trennt,
- die organische Phase c1 teilweise als Rücklauf in der Destillationseinheit b2) und teilweise ohne weitere Waschung in eine destillative Aufreinigung g) des (Meth)acrylsäureesters leitet und
- die wässrigen Phasen c2) und c3) zumindest teilweise in eine Alkoholrückgewinnung d) leitet.

2. Verfahren nach Anspruch 1, **dadurch gekennzeichnet, daß** man in der Alkoholrückgewinnung d) aus den wässrigen Phasen c2) und c3) eine im wesentlichen (Meth)acrylsäureester und Alkohol enthaltende Fraktion d1) abtrennt und diese zumindest teilweise in die Reaktionszone b) führt.

3. Verfahren nach Anspruch 1 oder 2, **dadurch gekennzeichnet, daß** man in der Alkoholrückgewinnung d) der wässrigen Phasen c2) und c3) eine im wesentlichen Wasser enthaltene Fraktion d2) abtrennt und diese zumindest teilweise als Waschflüssigkeit im Waschschritt c) verwendet.

4. Verfahren nach einem der Ansprüche 1 bis 3, **dadurch gekennzeichnet, daß** man in der Alkoholrückgewinnung d) der wässrigen Phasen c2) und c3) eine Michael-Additionsprodukt enthaltende Fraktion d3) abtrennt und diese in die Reaktionszone b) führt.

5. Verfahren nach einem der Ansprüche 1 bis 4, **dadurch gekennzeichnet, daß** vor der destillativen Aufreinigung g) des (Meth)acrylsäureesters eine Leichtsiederabtrennung e) durchlaufen wird, in der man eine im wesentlichen (Meth)acrylsäureester enthaltende Fraktion e3) von einer im wesentlichen Wasser, Essigsäurealkylester, Ether und (Meth)acrylsäureester enthaltenden Fraktion trennt.

6. Verfahren nach Anspruch 5, **dadurch gekennzeichnet, daß** man die im wesentlichen Wasser, Essigsäurealkylester, Ether. und (Meth)acrylsäureester enthaltende Fraktion in eine organische Phase e1), die im wesentlichen Essigsäurealkylester, Ether und (Meth)acrylsäureester enthält und eine wässrige Phase e2) trennt.

7. Verfahren nach Anspruch 6, **dadurch gekennzeichnet, daß** man die wässrige Phase e2) zumindest teilweise in die Alkoholrückgewinnung d) und/oder den Waschschritt c) führt.

8. Verfahren nach Anspruch 6 oder 7, **dadurch gekennzeichnet, daß** man die organische Phase e1) in eine Leichtsiederaufarbeitung f) führt und dort in eine im wesentlichen (Meth)acrylsäureester enthaltende Fraktion f2) und eine im wesentlichen Essigsäurealkylester und Ether enthaltende Fraktion f1) auftrennt.

9. Verfahren nach einem der Ansprüche 1 bis 8, **dadurch gekennzeichnet, daß** man die Fraktion e3) in eine Reindestillation g) leitet und dort in eine (Meth)acrylsäureester in einer Reinheit von mindestens 99,9 Gew% enthaltende Fraktion g1) und eine (Meth)acrylsäureester und Michael-Additionsprodukt enthaltende Fraktion g2) auftrennt.

10. Verfahren nach Anspruch 8 oder 9, **dadurch gekennzeichnet, daß** man die Fraktion f2) in die Leichtsiederabtrennung e) leitet und/oder als Rücklauf in die Destillationseinheit b2) verwendet.

11. Verfahren nach Anspruch 9 oder 10, **dadurch gekennzeichnet, daß** man die Fraktion g2) zumindest teilweise in die Reaktionszone b) leitet.

12. Verfahren nach einem der Ansprüche 1 bis 11, **dadurch gekennzeichnet, daß** es sich bei der eingesetzten (Meth)acrylsäure um vorbehandelte Roh-(Meth)acrylsäure handelt.

13. Verfahren nach Anspruch 12, **dadurch gekennzeichnet, daß** man die Roh-(Meth)acrylsäure vor der Veresterung zunächst in einer Vorbehandlung a) in Gegenwart eines Amins, Hydrazins oder Hydrazinderivats behandelt. .

14. Verfahren nach einem der Ansprüche 2 bis 13, **dadurch gekennzeichnet, daß** man den zugeführten Alkohol nicht in die Reaktionszone b) leitet, sondern als Rücklauf in der Alkoholrückgewinnung d) verwendet und die Fraktion d1) in die Reaktionszone b) führt.

15. Verfahren nach Anspruch 14, **dadurch gekennzeichnet, daß** man die Fraktion d1) gasförmig in die Reaktionszone b) führt.

16. Verfahren nach einem der vorstehenden Ansprüche, **dadurch gekennzeichnet, daß** man als Alkohol Methanol oder Ethanol und als (Meth)acrylsäure Acrylsäure verwendet.

## Claims

1. A process for the preparation of (meth)acrylates by reacting (meth)acrylic acid having a carbonyl content of less than 50 ppm with an alcohol in the presence of at least one acidic catalyst and at least one polymerization inhibitor in a reaction zone b) consisting of at least one reactor b1) having at least one connected distillation unit b2), wherein
- in the reactor b1) the temperature is brought to from 120 to 150°C and
- the desired (meth)acrylic acid : alcohol stoichiometry is established,
- the distillate of the distillation unit b2) is treated with a wash liquid (wash step c)),
- the discharge from the wash step c) is separated into at least one organic phase c1) and at least two aqueous phases c2) and c3),
- some of the organic phase c1) is passed as reflux to the distillation unit b2) and some of it is passed without further washing to a distillative purification g) of the (meth)acrylate and
- the aqueous phases c2) and c3) are passed at least partly to an alcohol recovery d).

2. The process according to claim 1, wherein, in the alcohol recovery d), a fraction d1) comprising substantially (meth)acrylate and alcohol is separated from the aqueous phases c2) and c3) and said fraction is fed at least partly into the reaction zone b).

3. The process according to claim 1 or 2, wherein, in the alcohol recovery d) of the aqueous phases c2) and c3), a substantially water-comprising fraction d2) is separated off and said fraction is used at least partly as wash liquid in the wash step c).

4. The process according to any of claims 1 to 3, wherein, in the alcohol recovery d) of the aqueous phases c2) and c3), a fraction d3) comprising Michael adducts is separated off and said fraction is fed into the reaction zone b).

5. The process according to any of claims 1 to 4, wherein, before the distillative purification g) of the (meth)acrylate, a low boiler removal e) is carried out, in which a substantially (meth)acrylate-comprising fraction e3) is separated from a fraction comprising substantially water, alkyl acetate, ether and (meth)acrylate.

6. The process according to claim 5, wherein the fraction comprising substantially water, alkyl acetate, ether and (meth)acrylate is separated into an organic phase e1), which substantially comprises alkyl acetate, ether and (meth)acrylate, and an aqueous phase e2).

7. The process according to claim 6, wherein the aqueous phase e2) is fed at least partly to the alcohol recovery d) and/or to the wash step c).

8. The process according to claim 6 or 7, wherein the organic phase e1) is fed to a low boiler working-up f) and is separated there into a substantially (meth)acrylate-comprising fraction f2) and a fraction f1) comprising substantially alkyl acetate and ether.

9. The process according to claims 1 to 8, wherein the fraction e3) is passed into a purifying distillation g) and is separated there into a fraction g1) comprising (meth)acrylate in a purity of at least 99.9% by weight and a fraction g2) comprising (meth)acrylate and Michael adduct.

10. The process according to claim 8 or 9, wherein the fraction f2) is passed into the lower boiler removal e) and/or is used as reflux into the distillation unit b2).

11. The process according to claim 9 or 10, wherein the fraction g2) is passed at least partly into the reaction zone b).

12. The process according to any of claims 1 to 11, wherein the (meth)acrylic acid used is pretreated crude (meth)acrylic acid.

13. The process according to claim 12, wherein the crude (meth)acrylic acid is first treated in a pretreatment a) in the presence of an amine, hydrazine or hydrazine derivative before the esterification.

14. The process according to any of claims 2 to 13, wherein the alcohol fed in is not passed into the reaction zone b) but is used as reflux in the alcohol recovery d) and the fraction d1) is fed into the reaction zone b).

15. The process according to claim 14, wherein the fraction d1) is fed in gaseous form into the reaction zone b).

16. The process according to any of the preceding claims, wherein the alcohol used is methanol or ethanol and the (meth)acrylic acid used is acrylic acid.

## Revendications

1. Procédé de préparation d'esters d'acide (méth)acrylique par réaction d'acide (méth)acrylique présentant une teneur en carbonyle inférieure à 50 ppm avec un alcool, en présence d'au moins un catalyseur acide et d'au moins un inhibiteur de polymérisation, dans une zone réactionnelle b) constituée d'au moins un réacteur b1) avec au moins une unité de distillation b2) raccordée, **caractérisé en ce que**
- dans le réacteur b1), on ajuste la température à 120 jusqu'à 150°C et
- la stoechiométrie souhaitée acide (méth)acrylique/alcool,
- on traite le distillat de l'unité de distillation b2) avec un liquide de lavage [étape de lavage c)],
- on sépare l'effluent de l'étape de lavage c) en au moins une phase organique c1) et au moins deux phases aqueuses c2) et c3),
- on conduit la phase organique c1) partiellement sous forme de reflux dans l'unité de distillation b2) et partiellement, sans autre lavage, dans une purification par distillation g) de l'ester d'acide (méth)acrylique, et
- on conduit les phases aqueuses c2) et c3) au moins partiellement dans une récupération d'alcool d).

2. Procédé suivant la revendication 1, **caractérisé en ce que**, dans la récupération d'alcool d), on isole à partir des phases aqueuses c2) et c3) une fraction d1) contenant essentiellement de l'ester d'acide (méth)acrylique et de l'alcool et on conduit celle-ci au moins partiellement dans la zone réactionnelle b).

3. Procédé suivant la revendication 1 ou 2, **caractérisé en ce que**, dans la récupération d'alcool d), on isole à partir des phases aqueuses c2) et c3) une fraction d2) contenant essentiellement de l'eau et on utilise celle-ci au moins partiellement comme liquide de lavage dans l'étape de lavage c).

4. Procédé suivant l'une des revendications 1 à 3, **caractérisé en ce que**, dans la récupération d'alcool d), on isole à partir des phases aqueuses c2) et c3) une fraction d3) contenant un produit d'addition de Michael et on conduit celle-ci dans la zone réactionnelle b).

5. Procédé suivant l'une des revendications 1 à 4, **caractérisé en ce que**, avant la purification par distillation g) de l'ester d'acide (méth)acrylique, on passe par une séparation des substances à bas point d'ébullition e), dans laquelle on sépare une fraction e3) contenant essentiellement de l'ester d'acide (méth)acrylique d'une fraction contenant essentiellement de l'eau, de l'acétate d'alkyle, de l'éther et de l'ester d'acide (mëth)acrylique.

6. Procédé suivant la revendication 5, **caractérisé en ce qu'**on sépare la fraction contenant essentiellement de l'eau, de l'acétate d'alkyle, de l'éther et de l'ester d'acide (méth)acrylique en une phase organique e1), qui contient essentiellement de l'acétate d'alkyle, de l'éther et de l'ester d'acide (mêth)acrylique, et une phase aqueuse e2).

7. Procédé suivant la revendication 6, **caractérisé en ce qu'**on conduit la phase aqueuse e2) au moins partiellement dans la récupération d'alcool d) et/ou l'étape de lavage c).

8. Procédé suivant la revendication 6 ou 7, **caractérisé en ce qu'**on conduit la phase organique e1) dans un traitement de substances à bas point d'ébullition f) et là on la sépare en une fraction f2) contenant essentiellement de l'ester d'acide (méth)acrylique et une fraction f1) contenant essentiellement de l'acétate d'alkyle et de l'éther.

9. Procédé suivant l'une des revendications 1 à 8, **caractérisé en ce qu'**on conduit la fraction e3) dans une distillation à l'état pur g) et là on la sépare en une fraction g1) contenant de l'ester d'acide (méth)acrylique à une pureté d'au moins 99,9 % en poids et en une fraction g2) contenant de l'ester d'acide (méth)acrylique et du produit d'addition de Michael.

10. Procédé suivant la revendication 8 ou 9, **caractérisé en ce qu'**on conduit la fraction f2) dans la séparation des substances à bas point d'ébullition e) et/ou on l'utilise comme reflux dans l'unité de distillation b2).

11. Procédé suivant la revendication 9 ou 10, **caractérisé en ce qu'**on conduit la fraction g2) au moins partiellement dans la zone réactionnelle b).

12. Procédé suivant l'une des revendications 1 à 11, **caractérisé en ce que**, pour ce qui concerne l'acide (méth)acrylique mis en oeuvre, il s'agit d'acide (méth)acrylique brut prétraité.

13. Procédé suivant la revendication 12, **caractérisé en ce que**, avant l'estérification, on traite l'acide (méth)acrylique brut tout d'abord dans un prétraitement a), en présence d'une amine, d'une hydrazine ou d'un dérivé d'hydrazine.

14. Procédé suivant l'une des revendications 2 à 13, **caractérisé en ce qu'**on ne conduit pas l'alcool amené dans la zone réactionnelle b), mais qu'on l'utilise comme reflux dans la récupération d'alcool d) et on conduit la fraction d1) dans la zone réactionnelle b).

15. Procédé suivant la revendication 14, **caractérisé en ce qu'**on conduit la fraction d1) dans la zone réactionnelle b) sous une forme gazeuse.

16. Procédé suivant l'une des revendications précédentes, **caractérisé en ce que**, comme alcool, on utilise du méthanol ou de l'éthanol et, comme acide (méth)acrylique, de l'acide acrylique.
